# EUROPEAN PATENT APPLICATION

(11) **EP 1 835 025 A1**
(43) Date of publication of application: **19.09.2007**
(21) Application number: 05825223.0
(22) Date of filing: 15.12.2005
(51) Int. Cl.: C12N 9/26, C12N 9/10, C07H 3/06

(54) **NOVEL ENZYME FOR THE PRODUCTION OF PREBIOTIC OLIGOSACCHARIDES**

(30) Priority: 16.12.2004 ES 200402994
(71) Applicant: Consejo Superior de Investigaciones Cientificas, 28006 Madrid (ES); UNIVERSIDAD AUTONOMA DE MADRID CIUDAD UNIVERSITARIA CANTOBLANCO, 38049 Madrid (ES)
(72) Inventor: FERNÁNDEZ LOBATO, María, E-28760 Tres Cantos (Madrid) (ES); MARÍN ALBERDI, Maria Dolores, Greater London NV6 6SX (GB); JIM NEZ MARTÍNEZ, A., Insto. Biologí Molecular, E-28049 Madrid (ES); PLOU GASCA, F.J., Inst Catálisis Y Petroleoquimica, E-28049 Madrid (ES); GÓMEZ DE SEGURA UGALDO, M.A., Insto. Catálisis, E-28049 Madrid (ES); ALCALDE GALEOTE, M., Insto. Catálisis, E-28049 Madrid (ES)
(74) Representative: Barlocci, Anna
(86) International application number: PCT/ES2005/070177
(87) International publication number: WO 2006/064078

(57) **Abstract**

An industrially viable process of obtaining prebiotic oligosaccharides using a new Xanthophyllomyces dendrorhous enzyme, **characterized by** showing α-glucosidase activity is provided. A process of obtaining an enzymatic product with α-glucosidase activity as well as the substantially pure enzyme with α-glucosidase activity, are also provided. The enzymatic product and the enzyme have a high action spectrum and a high specific activity as positive aspects. Prebiotic oligosaccharides are used in nutrition.

## Description

This invention is related to the field of the biotechnological industry and, specifically, to the food and agriculture field dedicated to obtaining prebiotic oligosaccharides to be used as functional ingredients in dietetic products, dairy products, children's foods and animal foods. It is also related to the field of the pharmaceutical and cosmetics industry.

### BACKGROUND ART

Carbohydrates, the most abundant biological material in nature, are used as starting elements in a wide variety of industrial processes; as a result, the enzymes involved in their metabolism are of great interest from both the basic as well as the technological points of view.

The field of prebiotic oligosaccharides as functional ingredients in nutrition has grown considerably in recent years. The term prebiotic was introduced by Gibson and Roberfroid, who defined prebiotics as non-digestible ingredients of foods beneficially affecting the host by the selective stimulation of growth and/or activity of one or a limited group of bacteria in the colon. Prebiotic oligosaccharides are basically fructooligosaccharides (FOS), although isomaltooligosaccharides (IMOS) and transgalactooligosaccharides (GalOS) are also widely applicable. The effects of prebiotics on the person taking them may vary significantly: reduction of the episodes of diarrhea caused by rotavirus; improvement of lactose intolerance symptoms; control of constipation by a fecal mass increase; increased calcium absorption, and consequently a reduced risk of osteoporosis; decreased mutagenic capability of certain microbial enzymes associated with colon cancer, such as nitro-reductase; possible reduction of diseases related to dyslipidemia, etc.

Isomaltooligosaccharides (IMOS) are sugars formed by glucose units bound by means of α-1,6 bonds, although products which are marketed also contain oligosaccharides formed by glucose units bound by α-1,6 and α-1,4 bonds, such as the trisaccharide panose. The interest in IMOS is based on their prebiotic properties, in addition to improving the hygroscopic and rheological characteristics of the products in which they are incorporated. Fortunately these interactions are very selective: IMOS are specifically metabolized by the beneficial bacterial microflora (Bifidobacterium, Bacteroides, Lactobacillus genres), favoring growth of these microorganisms, showing no type of interaction with the potentially pathogenic microflora (bifidus effect). They are also applicable for preventing cavities (as they inhibit glucan synthesis on the part of Streptococcus mutans and related species) and as immunostimulating substances.

Maltooligosaccharides are sugars which typically have between 2 and 7 glucose units bound by means of α-1,4 bonds ([α-D-Glu-(1→4)-]₂₋₇). Their ingestion improves the condition of the colon in humans; consuming syrups rich in maltotetraose ([α-D-Glu-(1→4)-]₄) reduces intestinal levels of Clostridium perfringens and members of the Enterobacteriaceae family. However, and given that they are usually hydrolyzed by digestive enzymes after their ingestion, they cannot be strictly considered prebiotics. Maltooligosaccharides have a series of properties (high solubility, high viscosity, low sweetening power, they are not hygroscopic, etc.) which provide them with a large number of applications in nutrition. Among these applications their use as binders, fat substitutes, texturizing agents or thickeners must be pointed out (cf. F. Barresi et al., "Maltooligosaccharides from corn", Oligosaccharides in food and agriculture, G. Eggleston and G.L. Cote, eds., ACS Symposium Series, 2003, vol. 849, pp. 182-95).

Prebiotic oligosaccharides are commonly obtained at the industrial level by enzymatic synthesis, using glycosyltransferases or glycosidases. IMOS are produced from starch using two enzymatic steps. In the first step, the starch is partially degraded by the action of an α-amylase, obtaining maltooligosaccharides. In the second step, the maltooligosaccharides are converted into isomaltooligosaccharides by the combined action of a beta-amylase, which degrades the formed maltodextrins to maltose, and an α-glucosidase, which forms α-1,6 bonds by transfer (cf. T. Kaneko et al. "Effects of isomaltooligosaccharides with different degrees of polymerization on human fecal bifidobacteria", Biosci. Biotech. Biochem. 1994, vol. 58, pp. 2288-90). Enzymes from Aspergillus niger and Bacillus stearothermophilus are currently used for obtaining prebiotic oligosaccharides (cf. K.J. Duan et al., "Transglucosylation of a fungal α-glucosidase - The enzyme properties and correlation of isomaltooligosaccharide production", Ann. New York Acad. Sci. 1995, vol. 750, pp. 325-8; S. Mala et al., "Towards regioselective synthesis of oligosaccharides by use of α-glucosidases with different substrate specificity", Carbohydr. Res. 1999, vol. 322, pp. 209-18, respectively).

Annual prebiotic oligosaccharide production exceeds 10,000 tons and takes place mainly in Japan. A typical commercial product (Isomalto-900), manufactured by Showa Sangyo Co., is a syrup containing 75% (w/v) solids, more than 85% of which corresponds to oligosaccharides (cf. R.G. Crittenden et al., "Production and applications of food-grade oligosaccharides" Trends Food Sci. Technol. 1996, vol. 7, pp. 353-61). Given the industrial importance of prebiotic oligosaccharides, it is desirable to provide enzymes and processes for obtaining them that are industrially viable.

### DESCRIPTION OF THE INVENTION

The inventors have found a new Xanthophyllomyces dendrorhous enzyme characterized by showing α-glucosidase activity and useful for obtaining oligosaccharides. Thus, one aspect of the invention relates to a process of obtaining an enzymatic product with α-glucosidase activity which comprises culturing cells of X. dendrorhous in an appropriate medium and under appropriate conditions. A person skilled in the art will choose the culture medium and the conditions, such as pH, temperature and stirring for culturing X. dendrorhous by means of conventional methods. Culture examples are described in detail below.

An α-glucosidase (EC 3.2.1.20, α-D-glucoside glycosyl-hydrolase, according to the IUBMB Enzyme Nomenclature, CAS Registry Number 9001-42-7) acts on the non-reducer ends of a varied number of carbohydrates producing the successive release of D-glucose units.

The crude enzymatic product resulting from the process of the invention can now be used industrially to obtain oligosaccharides without requiring subsequent separation or purification steps. In any manner, in a particular embodiment of the invention the process further comprises the step of recovering the enzymatic product from the culture medium and/or from the cells, because the enzyme object of the invention is released extracellularly. Thus both the suspension of X. dendrorhous cells with the suitable culture medium so that the α-glucosidase activity has been expressed as well as the cell-free fraction are understood as the enzymatic product in this description. A person skilled in the art will choose the starting enzymatic product most suitable for each industrial process, i.e. crude or with a higher or lower purification level, by means of conventional methods.

In another particular embodiment of the invention, the X. dendrorhous cells belong to a strain selected from the group consisting of ATCC:MYA-131, ATCC 24230, CECT 11028 and CECT 1690 (CECT corresponds to the Colección Española de Cultivos Tipo *(Spanish Type Culture Collection)* in Burjassot, Valencia). A detailed description of the X. dendrorhous organism is included below. The ATCC: MYA-131 strain has been deposited in the American Type Culture Collection (12301 Parklawn Drive, Rockville, Md. 20852, USA). It was designated as UCD67-210 and catalogued as ATCC: MYA-131.

Another aspect of the invention relates to the enzymatic product with α-glucosidase activity obtainable by means of the process hereinbefore defined. The enzymatic product of the invention is very efficient in degrading maltose and oligosaccharides with α-1,4 bonds (e.g. maltotriose, maltoheptose) and also acts on polysaccharides such as maltodextrins and soluble starch. In a particular embodiment of the invention the enzymatic product of the invention is characterized in that the α-glucosidase activity has low substrate specificity, acting on maltose, maltotriose, maltoheptose, dextrins, X-α-glucoside, glycogen and soluble starch. In another particular embodiment of the invention, the enzymatic product does not have α-glucosidase activity on isomaltose, isomaltotriose, pullulan and dextran. In another particular embodiment, the α-glucosidase activity of the enzymatic product has a maximum value in the pH interval between 4.5 and 6.0 at 42 °C, and in a temperature interval between 40 and 50 °C.

The formation of a new glycosidic bond, which takes place in a transfer reaction (transglycosylation), and the hydrolysis of this same bond are two variants of the same catalytic process. Thus, some glycosidase enzymes are capable of catalyzing both the formation of a glycosidic bond as well as the hydrolysis thereof, and their activity in both directions greatly depends on the physiological conditions in which they act in vivo, or on the thermodynamic or kinetic control exercised in vitro. Glycosyltransferases (EC 2.4) are enzymes which catalyzes the transfer of sugar units from activated donor molecules to specific acceptor molecules, forming glycosidic bonds.

The inventors have found that in addition to the α-glucosidase activity, the enzymatic product of the invention has glycosyltransferase activity in the presence of one or several glucidic substrates, particularly maltooligosaccharides (e.g. maltose at high concentrations). In a particular embodiment the products resulting from the glycosyltransferase activity are oligosaccharides with α-1,4 bonds, particularly maltotriose and maltotetraose; oligosaccharides with α-1,6 bonds, particularly isomaltose; and/or mixed oligosaccharides with α-1,4 and α-1,6 bonds, particularly panose and the tetrasaccharide α-D-Glu-(1→6)-a-D-Glu-(1→4)-α-D-Glu-(1→4)-α-D-Glu.

Another aspect of the invention relates to a process of obtaining a substantially pure enzyme with α-glucosidase activity, which comprises the steps of: (a) obtaining an enzymatic product with α-glucosidase activity by means of culturing X. dendrorhous cells in an appropriate medium and under appropriate conditions; (b) recovering the enzymatic product from the culture medium and/or from the cells; and (c) purifying the enzymatic product until obtaining a substantially pure enzyme with α-glucosidase activity. In a particular embodiment of the invention, the X. dendrorhous cells belong to a strain selected from the group consisting of ATCC:MYA-131, ATCC 24230, CECT 11028 and CECT 1690. The invention also relates to a substantially pure enzyme with α-glucosidase activity obtainable by means of the defined process. Conventional purification methods can be used to obtain the enzyme of the invention. An example of a purification method is described in detail in this description.

The indicated substrate specificity features for the enzymatic product of the invention are also attributed to the purified enzyme. Glycosyltransferase activity is also characteristic of the purified enzyme. Furthermore, in a particular embodiment the enzyme is characterized by having a molecular weight of about 115 kDa determined by molecular filtration, and an isoelectric point of about 5.5. The inventors have characterized the purified enzyme kinetically and by mass spectrometry. These data is included below in this description.

Some positive aspects of the enzymatic product and of the enzyme of the invention are that they have a high action spectrum and a high specific activity, making them suitable candidates for hydrolyzing or modifying oligosaccharides. Another important aspect on the industrial level is that the enzymatic product and the enzyme are stable for long reaction times (e.g. 300 h) at a temperature of about 40 °C, as is illustrated in the detailed description below.

The present invention involves an industrially viable process of obtaining oligosaccharides. Thus, another aspect of the invention relates to a process of obtaining oligosaccharides which comprises allowing the previously defined enzymatic product or purified enzyme to act on one or several glucidic substrates. An expert in the art will choose the culture mediums, substrates and reaction conditions for carrying out the process by means of conventional methods. The enzyme or X. dendrorhous cells producing the enzyme, can furthermore be used as such or immobilized, physically or chemically coupled to a carrier material. The reuse of the enzyme or cells is thus allowed. Preparation examples are included below in this description.

Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following detailed description, examples and drawings are provided by way of illustration and are not intended to be limiting of the present invention.

### DESCRIPTION OF THE DRAWINGS

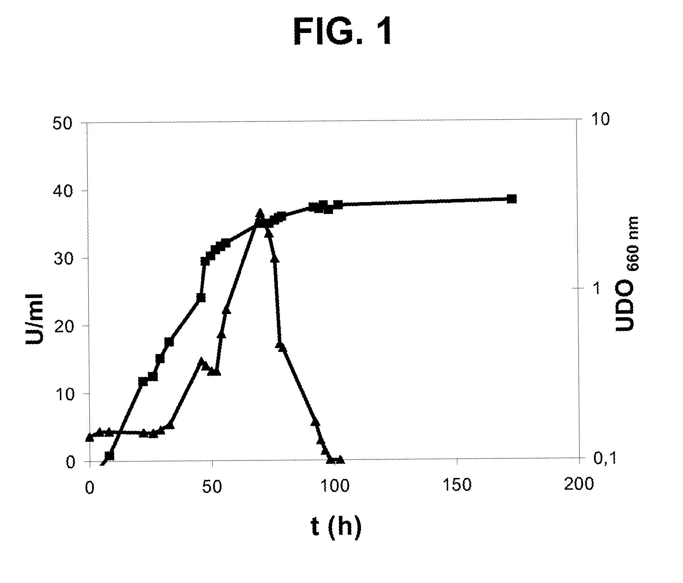

FIG. 1 shows the production of extracellular α-glucosidase activity throughout the X. dendrorhous culture. The yeast was grown in maltose minimal medium at 24 °C and constant orbital shaking at 160 rpm for 175 hours. Growth of the culture in ODU₆₆₀ₙₘ (squares) and α-glucosidase activity assessed in the extracellular medium in U/ml (triangles), reached at the times indicated, are represented. The activity was tested on 1% maltose.

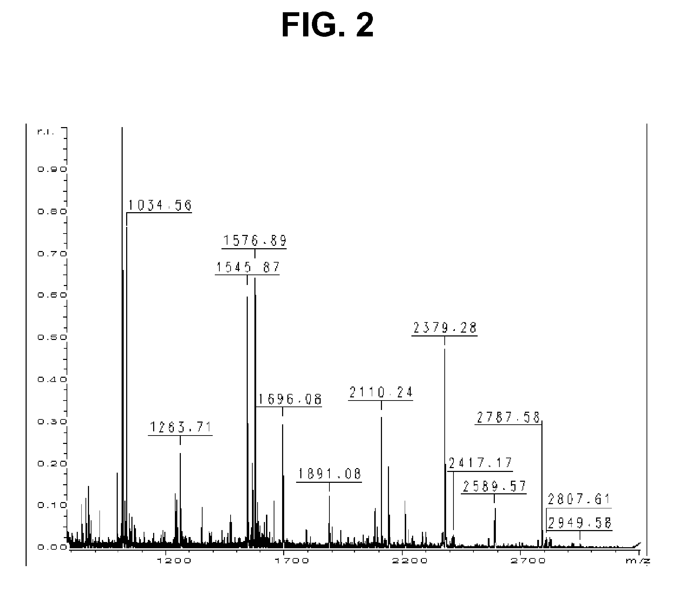

FIG. 2 shows the result of the MALDI-TOF mass spectrometry analysis of the protein purified to homogeneity. The relative intensity (r.i.) of the ionized tryptic peptides against the mass/charge (m/z) thereof is represented. The supernatant of digestion with trypsin was analyzed in a Bruker Autoflex MALDI-TOF mass spectrometer equipped with a reflector, using HCCA (α-cyano-4-hydroxycinnamic acid) as a matrix in saturation conditions.

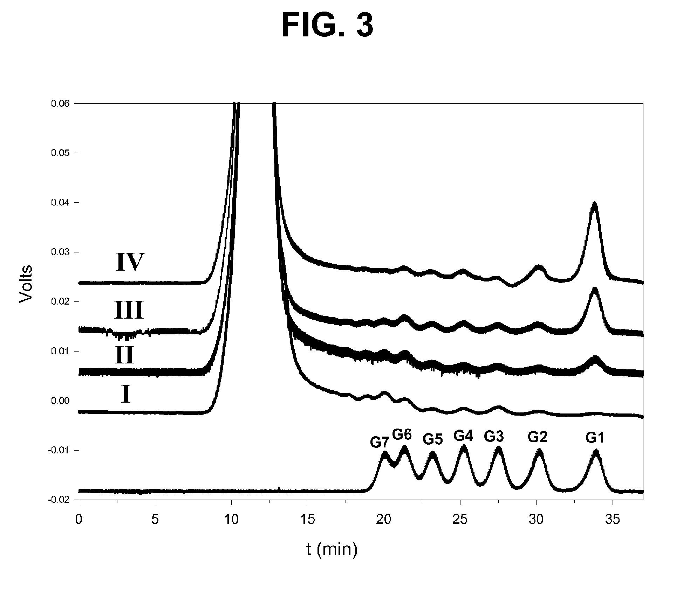

FIG. 3 shows the activity of the X. dendrorhous F-3 fraction on starch (Paselli SA2, Avebe, with a mean polymerization degree of 50 units of glucose). The tests were conducted using purified protein and a substrate concentration of 10% (w/v) in 0.2 M sodium acetate buffer, pH 5.4. A chromatogram of a mixture of oligosaccharides from G1 (glucose) to G7 (maltoheptose) is shown. The starch hydrolysis reaction chromatograms correspond to the following reaction times: I, time 0; II, 15 hours; III, 63 hours; IV, 168 hours.

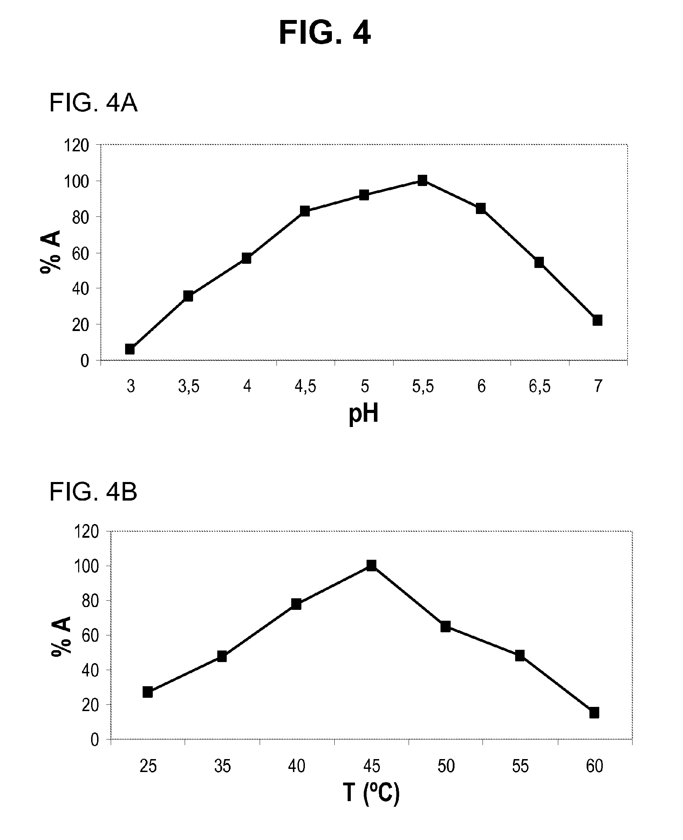

FIG. 4 indicates enzymatic activity (A) according to the pH and temperature, showing the corresponding maximum values. FIG. 4A: α-glucosidase activity was assessed at different pH values (pH of 3-7) and temperatures (25-60 °C). The test was carried out on maltose using a pure protein solution. 100% corresponds with an activity of 100 U/ml. A testing temperature of 42 °C and citrate or phosphate buffer (50 mM) for the pH intervals of 3-4.5 and 5-7 were used, respectively, in the pH assessment. FIG. 4B: the temperature test was carried out in 50 mM sodium phosphate, pH 5.5.

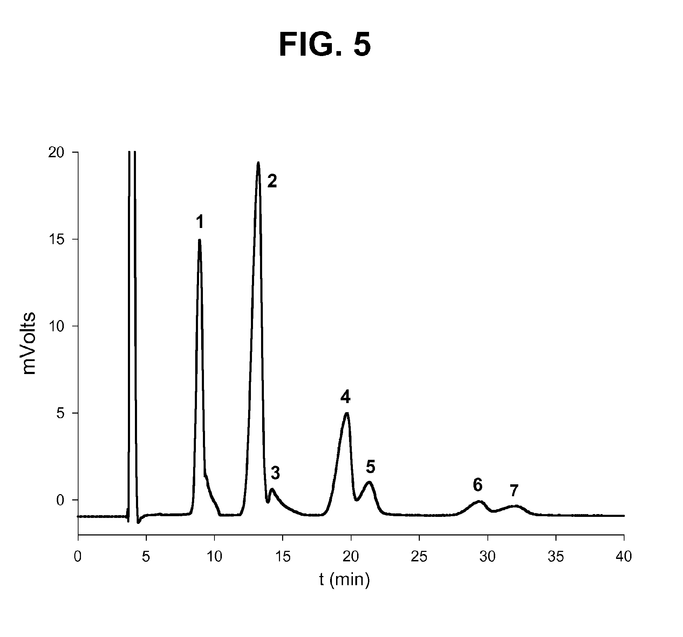

FIG. 5 shows the profile of products obtained after incubating maltose with the X. dendrorhous purified enzyme (F-3). The reaction conditions, HPLC analysis conditions and names of the compounds are the same as in TABLE 5. The analysis corresponds to 143 hours of reaction.

### DESCRIPTION OF XANTHOPHYLLOMYCES DENDRORHOUS

The yeast Xanthophyllomyces dendrorhous (also called Phaffia rhodozyma) is currently used in the biotechnological industry to produce astaxanthin, a carotenoid which has shown great effectiveness in the pigmentation of salmonids, crustaceans and in poultry farming. X. dendrorhous accumulates this carotene naturally and furthermore freely, which facilitates preparing the pigment. This makes it the microorganism in which production is globally more profitable than in any other. It is currently marketed as "Natupink" by Gist-Brocades (cf. EP 551676 B1). Hoffman-La Roche (Basel, Switzerland) has marketed the synthetic astaxanthin called "Carophyll pink". Antibióticos S.A. has developed the process for producing astaxanthin by means of fermenting selected strains of X. dendrorhous (cf. ES 2203315 A1).

The commercial interest in astaxanthin has generated a considerable advancement in the genetic and molecular knowledge of the producer organism. The genes involved in producing astaxanthin have been characterized, but some of the genes related to the use of carbon sources, such as those responsible for the synthesis of an endo-beta-1,3(4)-glucanase, a protease (cf. M.L. Bang et al., "Cloning and characterization of an endo-beta-1,3(4)-glucanase and aspartic protease from Phaffia rhodozyma CBS 6938", Appl. Microbiol. Biotechnol. 1999, vol. 51, pp. 215-22), or glyceraldehyde 3-phosphate dehydrogenase (cf. J.C. Verdoes et al., "Molecular characterization of the glyceraldehyde-3-phosphate dehydrogenase gene of Phaffia rhodozyma", Yeast 1997, vol. 13, pp. 1231-42) have also been characterized. A 240 kDa extracellular protein with beta-amylase activity has been partially purified and characterized (cf. A. Diaz et al., "Production and partial characterization of a beta-amylase by Xanthophyllomyces dendrorhous", Lett. Appl. Microbiol. 2003, vol. 36, pp. 203-7). Some proteins, such as invertase associated to the cell fraction or urease, have also been studied in this organism (D.S. Persike et al., "Invertase and urease activities in the carotenogenic yeast Xanthophyllomyces dendrorhous (formerly Phaffia rhodozyma)", Bioresour Technol. 2002, vol. 82(1), pp. 79-85).

In addition to the production of astaxanthin, X. dendrorhous has been indicated for the production of neokestose (CAS Registry Number 3688-75-3), a prebiotic trisaccharide obtained from sucrose (cf. S.M. Kritzinger et al., "The effect of production parameters on the synthesis of the prebiotic trisaccharide, neokestose, by Xanthophyllomyces dendrorhous (Phaffia rhodozyma)", Enzyme and Microbial Technology 2003, vol. 32, pp. 728-37).

### ENZYMATIC CHARACTERIZATION

### Expression of X. dendrorhous α-glucosidase activity in solid mediums

The α-glucosidase activity of the yeast was tested in solid mediums using X-α-D-glucoside (5-bromo-4-chloro-3-indolyl-α-D-glucopyranoside, Glycosynth), an element containing a chromophoric group bound to D-glucose by means of a glycosidic bond. 20 ml plates containing minimal medium for yeasts were used: 0.7% YNB (Yeast Nitrogen Base w/o Amino acids, DIFCO) (w/v), 2% agar (w/v), 2% carbon source, supplemented with X-α-D-glucoside. The plates were inoculated with the MYA-131 X. dendrorhous strain. A positive microorganism growth was obtained when maltose, starch, glucose, mannose or fructose were used. Good α-glucosidase activity, the acquisition of a deep blue color by the microorganisms expressing this activity, in the presence of starch and maltose was detected.

### Expression of X. dendrorhous amylase activity.

The yeast was grown in different mediums supplemented with different carbon sources. This organism can use different monosaccharides (glucose, mannose, and fructose), and oligosaccharides (maltose, maltotriose and sucrose). X. dendrorhous amylolytic activity was initially tested in solid medium using minimal mediums with starch (MMS): 0.7% YNB (Yeast Nitrogen Base w/o Amino acids, DIFCO) (w/v), 2% starch, 2% agar. Starch is soluble at room temperature but it precipitates at 4 °C. When a microorganism expresses amylase activity the starch concentration decreases around its colonies and precipitation does not occur; a hydrolysis halo appears. The polysaccharide hydrolysis halo was obtained with the X. dendrorhous strains ATCC 24230, CECT 11028, CECT 1690 and ATCC: MYA-131.

### Characterization of X. dendrorhous α-glucosidase activity after the culture and centrifugation of the cell-free fraction

The production of α-glucosidase activity was analyzed in X. dendrorhous cultures grown in minimal medium for yeasts: 0.7% YNB (Yeast Nitrogen Base w/o Amino acids, DIFCO) (w/v), 2% carbon source. The cultures were carried out in glass flasks incubated at 24 °C and with constant stirring at 160 rpm.

The cell-free fraction was obtained by centrifugation (F-0) and α-glucosidase activity in this fraction was tested, assessing the release of glucose on different substrates. A colorimetric test and standard methodology were used. The released glucose was quantified using the glucose oxidase-peroxidase coupled reaction: 0.4 ml of the solution to be assessed was mixed with 0.1 ml of A:B solution (20:1) (A: 0.85 U/ml glucose oxidase, 0.40 U/ml peroxidase in sodium phosphate buffer pH 5; B: 0.6% O-dianisidine). It was incubated for 30 minutes at 37 °C and spectrophotometrically quantified at 450 nm. A standard glucose curve (1 to 100 µg/ml) was used. The α-glucosidase activity unit is defined as the amount of enzyme required to release 20 µg/ml of glucose under the described conditions. FIG. 1 shows the results obtained when the MYA-131 strain is used and the test is performed on maltose. The test results are also shown in TABLE 1. Activity was not detected when dextran, isomaltose and isomaltotriose were used as a substrate.

**TABLE 1. α-glucosidase activity of the X. dendrorhous extracellular fraction on different glucose polymers.**

| Substrate | Specific activity (U/µg) |
|---|---|
| maltose | 30 |
| maltotriose | 18 |
| maltoheptose | 24 |
| maltodextrin | 21 |
| starch | 23 |

### Purification of the X. dendrorhous enzyme with α-glucosidase activity

The following method was used in the purification of the enzyme and the results are summarized in TABLE 2.

1rst) Concentrating the extracellular fraction with maximal activity using a tangential filtration system (30 kDa filter) and dialysis against 20 mM sodium phosphate, pH 7 (buffer A) for 3 hours at a temperature of 4 °C (F-1).

2nd) lon exchange chromatography at pH 7. The sample was applied to a 10 ml DEAE-Sephacel ion exchange column equilibrated with buffer A. Elution was carried out using a 0 to 0.5 M NaCl gradient. The fraction eluted at 0.05 M salt was dialyzed against 20 mM sodium acetate pH 4.5 (buffer B) (F-2).

3rd) Ion exchange chromatography at pH 4.5. The sample was applied to the DEAE-Sephacel ion exchange column equilibrated with buffer B and eluted using 0.2 M NaCl (F-3).

**TABLE 2. Summary of the purification process of X. dendrorhous α-glucosidase activity and obtained yield. Enzymatic activity during the purification process was determined using maltose as a substrate.**

| | activity (total U) | protein (µg) | yield (%) | specific activity (U/µg) | degree purification |
|---|---|---|---|---|---|
| S.N. (F-0) | 37000 | 1600 | 100 | 23 | 1 |
| concentrate (F-1) | 32300 | 1235 | 87 | 26 | 1.1 |
| DEAE pH 7 (F-2) | 20525 | 457 | 55 | 45 | 19.5 |
| DEAE pH 4.5 (F-3) | 14715 | 14 | 40 | 1065 | 46.1 |

### Determination of the molecular weight of the X. dendrorhous enzyme with α-glucosidase activity by means of molecular filtration

Molecular exclusion chromatography was used to determine the molecular weight of the α-glucosidase activity, using a liquid chromatography system (HPLC). A 24 ml Superose 12 HR10/30 column (Pharmacia) equilibrated with 50 ml of 50 mM sodium phosphate pH 7, 0.15 M NaCl at a rate of 0.25 ml/min was used. 0.1 ml of an enzymatic preparation with a specific activity of 1x10⁶ U/mg on maltose was applied. 0.3 ml fractions were collected. The following were used as molecular weight markers: immunoglobulin G (160,000 Da), albumin (67,000 Da), beta-lactoglobulin (35,000 Da) and cytochrome C (12,400 Da) which eluted at 12, 13.2, 14.42 and 16.64 ml, respectively. The entire process was carried out at 4 °C. The α-glucosidase activity was tested on maltose using the hereinbefore described methodology. Activity was detected in the fraction eluted at 12.3 ml, corresponding to a protein with a molecular weight of 115 ± 5% kDa.

The α-glucosidase purified to homogeneity according to the described process has a molecular weight of 115 ± 5% kDa calculated by molecular filtration (HPLC and Superose 12 HR) and an isoelectric point (pl) of 5.5.

### Characterization of the X. dendrorhous enzyme with α-glucosidase activity by mass spectrometry

The protein was digested with Trypsin (Promega Trypsin-TPCK) under standard digestion conditions (cf. A. Shevchenko et al., "Mass spectrometric sequencing of proteins silver-stained polyacrylamide gels", Anal Chem. 1996 vol. 68(5), pp. 850-8). The supernatant of digestion with trypsin was analyzed in a Bruker Autoflex MALDI-TOF (matrix-assisted laser desorption ionization/time-of-flight) type mass spectrometer equipped with a non-linear reflector with a UV-nitrogen laser at 337 nm, with 3 nanosecond pulses, following the standard methodology using HCCA (α-cyano-4-hydroxycinnamic acid) as a matrix under saturation conditions and 0.1% trifluoroacetic acids and 33% acetonitrile (v/v). The obtained result is shown in FIG. 2. This spectrum was used as the "peptide fingerprint" for identifying proteins in databases using online search engines (Mascot, Profound), based on the relative intensity (r.i.) of ionized tryptic peptides against the mass/charge (m/z) thereof. The isolated protein is a new molecule that has never before been described, as no MALDI-TOF profiles coinciding with proteins known by their amino acid sequence or the corresponding sequence deduced by the DNA sequence, have been found.

### Characterization of the α-glucosidase activity of the purified enzyme

The hydrolytic activity of the enzyme purified to homogeneity (F-3) following the described process was tested on different substrates. The maximum activity level is obtained on maltose, maltoheptose, soluble starch (Difco or Sigma) and dextrins. No activity was observed on dextran, pullulan, isomaltose and isomaltotriose. The obtained results are shown in TABLE 3.

**TABLE 3. α-glucosidase activity of the X. dendrorhous F-3 fraction on different substrates. The tests were conducted using purified protein and a 1% concentration for all the tested substrates.**

| Substrate | Specific activity (U/µg) |
|---|---|
| maltose | 1172 |
| maltotriose | 647 |
| maltoheptose | 997 |
| maltodextrin | 747 |
| starch (Difco) | 947 |
| starch (Sigma) | 222 |
| glycogen | 197 |

The profile of products obtained in starch hydrolysis was studied by liquid chromatography. FIG. 3 clearly shows that the main final product formed in this reaction is glucose.

Glucosidase activity was tested at different pHs and temperatures. Maximum activity levels were obtained in a pH range comprised between 4.5 and 6 and a temperature of 40-50 °C. FIG. 4 shows the obtained results.

The kinetic characterization of the enzyme purified according to the described process was carried out on maltose, maltotriose, maltoheptose and starch. The data are included in TABLE 4. The tests were carried out in 30 minutes and with 100 ng of enzyme for each reaction. The molecular weight of the starch, supplied by Avebe, is 8100 Da.

**TABLE 4. Kinetic constants of X. dendrorhous α-glucosidase glycosylhydrolase activity.**

| Substrate | Vₘₐₓ (µg glucose min⁻¹) | K_{cat} (min⁻¹) | Km (mM) | K_{cat}/Kₘ (mM⁻¹ min⁻¹) |
|---|---|---|---|---|
| maltose | 23 | 460 | 2.71 | 170 |
| maltotriose | 12 | 240 | 0.55 | 436 |
| maltoheptos | 15 | 300 | 0.86 | 348 |
| starch | 4.29 | 85.8 | 0.88 | 97.5 |

### Glycosyltransferase activity of the X. dendrorhous enzyme

A study was carried out using a high concentration of maltose (240 g/l), conditions which can favor the formation of glycosidic bonds in detriment of hydrolysis. The profile of the formed products is shown in FIG. 5. It can be seen that the X. dendrorhous α-glucosidase exhibits transfer activity. Furthermore, two families of products are formed: oligosaccharides with α-1,4 bonds (maltooligosaccharides: maltotriose [α-D-Glu-(1→4)-α-D-Glu-(1→4)-α-D-Glu]; maltotetraose [α-D-Glu-(1→4)-α-D-Glu-(1→4)-α-D-Glu-(1→4)-α-D-Glu]) and oligosaccharides containing an α-1,6 bond (isomaltose [α-D-Glu-(1→6)-α-D-Glu], panose [α-D-Glu-(1→6)-α-D-Glu-(1→74 )-α-D-Glu] and the tetrasaccharide: α-D-Glu-(1→6)-α-D-Glu-(1→4)-a-D-Glu-(1→4)-α-D-Glu).

TABLE 5 shows the composition (in g/l) of the sugars assessed in the reaction mixture over 288 hours of incubation. The new enzyme characterized in this work continues maintaining glycosyltransferase activity after 288 hours at 40 °C.

TABLE 5. Composition of the reaction mixture over time after incubating maltose with the purified X. dendrorhous enzyme (F-3). Reaction conditions: 240 g maltose/I in 0.2 M sodium acetate (pH 5.4), 40 °C, 150 rpm. HPLC analysis using a Waters delta 500 pump, Lichrospher 1 00-NH2 column (Merck), 250 x 4.6 mm, 75:25 v/v acetonitrile:water, 0.7 ml/min, 25 °C, Varian refraction index detector. Name of the compounds: 1, glucose; 2, maltose [α-D-Glu-(1→4)-α-D-Glu]; 3, isomaltose [α-D-Glu-(1→6)-α-D-Glu]; 4, maltotriose [α-D-Glu-(1→4)-α-D-Glu-(1→4)-α-D-Glu]; 5, panose [α-D-Glu-(1→6)-α-D-Glu-(1→4)-α-D-Glu]; 6, maltotetraose (α-D-Glu-(1→4)-α-D-Glu-(1→4)-α-D-Glu-(1→4)-α-D-Glu); 7, the tetrasaccharide α-D-Glu-(1→6)-α-D-Glu-(1→4)-α-D-Glu-(1→4)-α-D-Glu.

| Reaction time (h) | Composition of the reaction mixture (grams/liter) | | | | | | |
|---|---|---|---|---|---|---|---|
| | 1 saccha | 2 | 3 | 4 | 5 | 6 | 7 |
| 0 | 0 | 240.0 | 0 | 0 | 0 | 0 | 0 |
| 27 | 24.6 | 183.4 | 0 | 26.3 | 5.7 | 0 | 0 |
| 117 | 47.2 | 103.0 | 12.7 | 42.6 | 14.7 | 11.0 | 8.8 |
| 143 | 51.6 | 94.4 | 12.4 | 42.8 | 16.1 | 12.4 | 10.1 |
| 216 | 60.9 | 78.1 | 13.1 | 39.3 | 18.9 | 15.1 | 14.5 |
| 288 | 66.3 | 69.2 | 14.4 | 35.9 | 21.2 | 15.4 | 17.6 |

### PREPARATION EXAMPLES

### EXAMPLE 1: Production of α-glucosidase throughout an X. dendrorhous culture growing in rich medium

X. dendrorhous of the ATCC 24230 strain grown in 100 ml of rich medium for yeasts (YEP) supplemented with maltose (YEPM): 1% yeast extract (DIFCO) (w/v), 2% bactopeptone (DIFCO) (w/v), 2% maltose (w/v), was cultured to produce α-glucosidase. The culture continued for 60 hours. Cell growth was spectrophotometrically assessed following the absorbance of the culture at an optical density of 660 nm (ODU₆₆₀). A 250 ml glass flask, a temperature of 24 °C and constant orbital shaking were used. The stationary phase was reached after 35 hours of growth at 5 ODU₆₆₀. 1 ml of culture was taken every 3-4 hours and the cells were removed by centrifugation for 2-3 minutes in a microcentrifuge (16000 x g).

### EXAMPLE 2: Use of the enzyme for producing glucose from maltose with supernatant of the rich medium

The culture supernatant of EXAMPLE 1 was used for the release of glucose from maltose due to the action of the glucosidase activity of the supernatant in which the extracellular enzyme is located. To that end, 0.5 ml of the cell-free fraction and 0.5 ml of 1% maltose in 50 mM sodium phosphate buffer pH 5.5 were mixed and incubated at 42 °C for 60 minutes. Considerable glucosidase activity levels were obtained from the end of the logarithmic growth phase of the cultures until the beginning of the stationary phase for about 20 hours of culture. Maximum activity levels (20 U/ml) were obtained at 3-4 ODU₆₆₀.

### EXAMPLE 3: Production of α-glucosidase throughout a X. dendrorhous culture grown in minimal medium with starch.

X. dendrorhous MYA-131 grown in 100 ml of minimal medium with starch (MMS): 0.7% YNB (Yeast Nitrogen Base w/o Amino acids, DIFCO) (w/v), 2% starch (w/v), DIFCO) was cultured to produce α-glucosidase, and the culture continued for 150 hours. Cell growth was carried out and assessed as in the previous example. The stationary phase was reached after 75 hours of growth, at 3.4 ODU₆₆₀. The cell-free fraction was obtained every 5-7 hours as in EXAMPLE 1.

### EXAMPLE 4: Use of the enzyme for producing glucose from maltose with supernatant of minimal medium with starch

The supernatant of the culture of EXAMPLE 3 was used for the release of glucose from maltose due to the action of glucosidase activity of the supernatant in which the extracellular enzyme is located. To that end, 0.5 ml of the cell-free fraction and 0.5 ml of 1% maltose in 50 mM sodium phosphate buffer, pH 5.5 were mixed and incubated at 42 °C for 60 minutes. Assessable glucosidase activity levels were obtained from half the logarithmic growth phase of the culture until the beginning of the stationary phase, for about 30 hours of culture. Maximum activity levels (15 U/ml) were obtained at 2.3-2.5 ODU₆₆₀.

### EXAMPLE 5: Degradation of starch from the extracellular enzyme of an X. dendrorhous culture grown in minimal medium with starch

The starch present in the medium throughout the growth curve of the culture of EXAMPLE 3 was quantified with Lugol's iodine (0.15% l₂ (w/v), 0.5% Kl (w/v)). To that end, 0.05 ml of the cell-free fraction was mixed with 0.05 ml of 50 mM sodium phosphate buffer, pH 5.5. 0.1 ml of Lugol's iodine and 2.5 ml of water were added to 0.025 ml of this mixture. The polysaccharide was spectrophotometrically quantified at 595 nm using a standard starch curve (1-10 mg/ml). The 100% starch value was that assessed in the initial culture medium which was used to carry out the inoculum. By using this methodology, a decrease of the starch in the extracellular medium for the first 30 hours of culture was not observed. 10, 20, 40 and 60% reductions of the polysaccharide concentration were obtained at 50, 60, 70 and 80 hours of culture, respectively. The last value, 60%, remained constant during prolonged growths of up to 300 hours.

### EXAMPLE 6: Degradation of starch catalyzed by X. dendrorhous α-glucosidase

A 10% solution (w/v) of soluble starch (Paselli SA2, with a mean polymerization degree of 50 glucose units) in 0.2 M sodium acetate buffer, pH 5.4, was prepared. 0.5 ml of a solution of pure α-glucosidase corresponding to fraction 3 of TABLE 2 were added to 10 ml of this previous solution. The reaction mixture was incubated for 168 hours at 40 °C in an orbital shaker at 200 rpm. The profile of products obtained in the starch hydrolysis was studied by liquid chromatography. The analysis of the formed products was carried out by means of HPLC using a Waters 515 pump, 2 Aminex HPX-42A columns (BioRad) with dimension of 300 x 4.6 mm, water as a mobile phase at 0.6 ml/min, 65 °C, and a Waters refraction index detector.

### EXAMPLE 7: Formation of oligosaccharides from maltose catalyzed by X. dendrorhous α-glucosidase

A solution with a high maltose concentration (240 g/l) in 0.2 M sodium acetate buffer, pH 5.4, was prepared. 0.7 µg of α-glucosidase from the pure solution of α-glucosidase corresponding to fraction 3 of TABLE 2 were added. The reaction mixture was incubated for 288 hours at 40 °C, with orbital shaking at 200 rpm. Aliquots were extracted at different times and incubated for 5 minutes at 80 °C to inactivate the enzyme. They were diluted to 1:4 (v/v) with water, centrifuged for 5 minutes at 6000 rpm in an Eppendorf tube with a 0.45 µm filter and analyzed by HPLC liquid chromatography. The profile of the formed products can be seen in FIG. 7. It can be seen that X. dendrorhous α-glucosidase exhibits transfer activity. Furthermore, two families of products are formed: oligosaccharides with α-1,4 bonds (maltooligosaccharides), oligosaccharides containing an α-1,6 bond (isomaltose, panose, etc.). After the time (288 hours) elapsed, the composition of the system was: 66.3 g/I of glucose, 69.2 g/I of maltose, 14.4 g/I of isomaltose, 35.9 g/I of maltotriose, 21.2 g/I of panose, 15.4 g/I of maltotetraose, and 17.6 g/I of the tetrasaccharide α-D-Giu-(1→6)-α-D-Glu-(1→4)-α-D-Glu-(1→4)-α-D-Glu.

## Claims

1. A process of obtaining an enzymatic product with α-glucosidase activity, which comprises culturing Xanthophyllomyces dendrorhous cells in an appropriate medium and under appropriate conditions.

2. The process according to claim 1, which further comprises the step of recovering the enzymatic product from the culture medium and/or from the cells.

3. The process according to any of claims 1 and 2, wherein the Xanthoiphyllomyces dendrorhous cells belong to a strain selected from the group consisting of ATCC:MYA-131, ATCC 24230, CECT 11028 and CECT 1690.

4. An enzymatic product with α-glucosidase activity obtainable by means of the process as defined in any of claims 1-3.

5. The enzymatic product according to claim 4, **characterized in that** the α-glucosidase activity has low substrate specificity, acting on maltose, maltotriose, maltoheptose, dextrins, X-α-glucoside, glycogen and soluble starch.

6. The enzymatic product according to any of claims 4 and 5, **characterized in that** it does not have α-glucosidase activity on isomaltose, isomaltotriose, pullulan and dextran.

7. The enzymatic product according to any of claims 4-6, wherein the α-glucosidase activity shows a maximum value in the pH interval between 4.5 and 6.0 at 42 °C, and in a temperature interval of 40 to 50 °C.

8. The enzymatic product according to any of claims 4-7, **characterized in that** it has glycosyltransferase activity in the presence of one or several glucidic substrates.

9. The enzymatic product according to claim 8, **characterized in that** the glucidic substrates are maltooligosaccharides.

10. The enzymatic product according to claim 9, wherein the products resulting from the glycosyltransferase activity are oligosaccharides with α-1,4 bonds, oligosaccharides with α-1,6 bonds and/or mixed oligosaccharides with α-1,4 and α-1,6 bonds.

11. The enzymatic product according to claim 10, wherein the products resulting from the glycosyltransferase activity are maltotriose, maltotetraose, isomaltose, panose and/or the tetrasaccharide α-D-Glu-(1→6)-α-D-Glu-(1→4)-α-D-Glu-(1→4)-α-D-Glu).

12. A process of obtaining oligosaccharides which comprises allowing the enzymatic product as defined in any of claims 4-11 to act on one or several glucidic substrates.

13. A process of obtaining an enzymatic product with α-glucosidase activity, according to any of the claims 2-3, which further comprises a step (c) of purifying the enzymatic product until obtaining a substantially pure enzyme.

14. A substantially pure enzyme with α-glucosidase activity obtainable by means of the process as defined in claim 13.

15. The enzyme according to claim 14, **characterized in that** the α-glucosidase activity has low substrate specificity, acting on maltose, maltotriose, maltoheptose, dextrins, X-α-glucoside, glycogen and soluble starch.

16. The enzyme according to any of claims 14 and 15, **characterized in that** it does not have α-glucosidase activity on isomaltose, isomaltotriose, pullulan and dextran.

17. The enzyme according to any of claims 14-16, wherein the α-glucosidase activity has a maximum value in the pH interval between 4.5 and 6.0 at 42 °C, and in a temperature interval between 40 and 50 °C.

18. The enzyme according to any of claims 14-17, **characterized in that** it has glycosyltransferase activity in the presence of one or several glucidic substrates.

19. The enzyme according to claim 18, **characterized in that** the glucidic substrates are maltooligosaccharides.

20. The enzyme according to claim 19, wherein the products resulting from the glycosyltransferase activity are oligosaccharides with α-1,4 bonds, oligosaccharides with α-1,6 bonds and/or mixed oligosaccharides with α-1,4 and α-1,6 bonds.

21. The enzyme according to claim 20, wherein the products resulting from the glycosyltransferase activity are maltotriose, maltotetraose, isomaltose, panose and/or the tetrasaccharide α-D-Glu-(1→6)-α-D-Glu-(1→4)-α-D-Glu-(1→4)-α-D-Glu).

22. The enzyme according to any of claims 14-21, **characterized by** having a molecular weight of about 115 kDa calculated by molecular filtration, and an isoelectric point of about 5.5.

23. A process of obtaining oligosaccharides which comprises allowing the enzyme defined in any of claims 14-22 to act on one or several glucidic substrates.
